# EUROPEAN PATENT APPLICATION

(11) **EP 2 992 815 A1**
(43) Date of publication of application: **09.03.2016**
(21) Application number: 14196955.0
(22) Date of filing: 09.12.2014
(51) Int. Cl.: A61B 5/00, A61B 5/01, G01K 1/14

(54) **Early detection electronic device of diabetic foot for the blood circulation**

(30) Priority: 02.09.2014 TW 103130213
(71) Applicant: Creative Sensor Inc., Taipei City 114 (TW)
(72) Inventor: Hsiao, Pin-Huang, 114 Neihu Dist., Taipei City (TW); Weng, Jen-Chun, 114 Neihu Dist., Taipei City (TW); Chen, Chih-Yang, 114 Neihu Dist., Taipei City (TW)
(74) Representative: 2K Patentanwälte Blasberg Kewitz & Reichel

(57) **Abstract**

An early detection electronic device (1, 5) of diabetic foot for lesion in the blood circulation detects foot temperatures and includes a base (10, 50), a first temperature detector (12, 12', 52, 52'), a second temperature detector (14, 54, 54'), and a microprocessor (16, 56). The base (10, 50) includes a platform part (100, 500), a first arranging part (102, 502), and a second arranging part (104, 504). The first arranging part (102, 502) is connected to the platform part (100, 500) and the second arranging part (104, 504). The first temperature detector (12, 12', 52, 52') is arranged on the first arranging part (102, 502) and detects foot temperature along a first axis (A1). The second temperature detector (14, 54, 54') is arranged on the second arranging part (104, 504) and detects foot temperature along a second axis (A2). There is an included angle between the first axis (A1) and the second axis (A2). The microprocessor (16, 56) receives the temperatures detected by the first temperature detector (12, 12', 52, 52') and the second temperature detector (14, 54, 54'), and generates a warning signal when the first temperature is lower than the second temperature.

## Description

### BACKGROUND OF THE INVENTION

### 1. Field of the Invention

The instant disclosure relates to a scientific detecting device; in particular, to an early detection electronic device of diabetic foot for lesion in blood circulation.

### 2. Description of Related Art

Diabetes mellitus is a life-time chronic disease resulted from the metabolism disorder of saccharide, protein and fat in human body. The diabetes mellitus could further result in heart and kidney complications. In recent years, as observed, the morbidity of diabetes mellitus is gradually raising year by year.

Among several complications resulted from diabetes mellitus, the diabetic foot is a rather serious chronic complication. The leading factor of the diabetic foot is the lesion of the sensorimotor or the lesion of the arterial vessel. However, no matter it results from the lesion of the sensorimotor, the lesion of the arterial vessel or both of them, it'll further result in the foot necrosis due to the bad blood circulation. Even more, the patient might need to have amputation.

Regarding to the diabetes mellitus, the lesion of the sensorimotor is majorly about the symptoms of the peripheral nerve. The symptoms of the peripheral nerve would affect the peripheral sensory nerves, motor nerves and autonomic nerves. In particular, the worse of all, it might make the patient unable to feel his feet and further lose the nerves self-protection via the sensory nerves.

The loss of self-protection is a kind of negative symptom, which damages part feeling function and protecting function of nerves, which is a main reason which the patient often have foot ulcer. Clinically, the patient can't notice the disorder of the peripheral nerves. Even many patients who have already lost nerves self-protection may think that there is no problem regarding to their peripheral nerves. Thus, it is not practical to make a diagnosis about whether the patient has lost his nerves self-protection.

The most advanced vessel photograph can provide the diagnosis about the foot blood circulation, but it is costly and is an invasive process. Thus, it can't be applied in the first -line screening. Therefore, how to provide a scientific detecting device which is for the early detection of diabetic foot and is also not invasive and economical becomes a problem waiting to be solved.

### SUMMARY OF THE INVENTION

Regarding to the prior art, the present invention is to provide an early detection electronic device of diabetic foot for lesion in blood circulation. The early detection electronic device of diabetic foot for lesion in blood circulation detects the temperatures of the patient's medial plantar and instep, so as to determine whether the patient has the initial symptoms of diabetic foot.

In order to achieve the goal above mentioned, the present invention provides an early detection electronic device of diabetic foot for lesion in blood circulation, which is used for detecting temperature of at least one foot. The early detection electronic device of diabetic foot for lesion in blood circulation comprises a base, at least one first temperature detector and at least one second temperature detector. The base comprises a platform part, at least one first arranging part and one second arranging part. The first arranging part is connected to the platform part and the second arranging part is connected to the first arranging part and parallel with the platform part. The first temperature detector is configured to be horizontally at a same height at the first arranging part, and to detect temperature of the foot along a first axis. The second temperature detector is configured to be on the second arranging part and to detect temperature of the foot along a second axis. Particularly, there is an included angle between the second axis and the first axis. For example, the included angle is 90 degrees, but it is not limited thereto.

The early detection electronic device of diabetic foot for lesion in blood circulation also comprises a microprocessor. The microprocessor is electrically connected to the first temperature detector and the second temperature detector. The microprocessor receives the temperatures detected by the first temperature detector and the second temperature detector. In particular, when the temperature of the foot along the first axis is lower than the temperature of the foot along the second axis, the microprocessor delivers a warning signal.

In one embodiment of the present invention, the base comprise two first arranging parts are respectively configured at two opposite sides of the base, and the second arranging parts (104 , 504) are connected to the first arranging parts. Two first temperature detectors are respectively disposed on the first arranging part. The first temperature detectors detect the temperature of the foot along the first axis and the opposite direction of the first axis respectively.

The early detection electronic device of diabetic foot for lesion in blood circulation also comprises an orientation recognizer. The orientation recognizer is configured on the first arranging part or the second arranging part. The orientation recognizer is electrically connected to the microprocessor. The orientation recognizer is used for recognizing whether one medial plantar of the foot is facing the first axis and driving the first temperature detector facing the first axis to start temperature detecting. In other words, the orientation recognizer is configured to recognize that the foot the patient puts on the platform part is his right foot or left foot, so as to prevent from measuring mistake. It should be noted that the orientation recognizer can also be configured on the second arranging part.

In another embodiment of the present invention, the first arranging part is connected to a central part of the platform part and the second arranging part so that the base (10, 50) is I-shaped. Two first temperature detectors are respectively configured on two opposite surfaces of the first arranging part. The first temperature detectors detect the foot temperature along the first axis and the direction opposite the first axis, respectively. Also, two second temperature detectors are respectively configured on the second arranging parts on two sides of the first arranging part. As a result, the temperatures of the patient's two feet can be detected respectively.

Moreover, the early detection electronic device of diabetic foot for lesion in blood circulation also comprises a displayer, a memory, a power supply unit, a wireless transmitter and an information input unit. The displayer is electrically connected to the microprocessor, the first temperature detector and the second temperature detector. The displayer not only can show the alarming signal delivered by the microprocessor but also can show the temperature detected by the first temperature detector and the second temperature detector. For example, the displayer is configured on the second arranging part, but it is not limited thereto.

The memory is electrically connected to the microprocessor, the first temperature detector and the second temperature detector, and is configured to save the temperatures detected by the first temperature detector and the second temperature detector. The power supply unit is electrically connected to the microprocessor and is configured to provide power for the operation of the early detection electronic device of diabetic foot for lesion in blood circulation. The wireless transmitter is electrically connected to the microprocessor. The wireless transmitter is configured to wirelessly transmit the temperatures detected by the first temperature detector and the second temperature detector to a monitoring and controlling system. The information input unit is electrically connected to the microprocessor for the patient to input the measurement information.

The present invention also provides a method for detecting diabetic foot, comprising steps as follows: 1) providing a base for putting at least one foot of the patient, at least one first temperature detector and at least one second temperature detector are configured on the base, and the first temperature detector detecting temperature of the medial plantar of foot and the second temperature detector detecting temperature of the instep; 2) determining whether the temperature of the medial plantar of foot is higher than the temperature of the instep; and 3) delivering an alarming signal when the temperature of the medial plantar of foot is lower than the temperature of the instep. Moreover, the plurality of the first temperature detectors and the plurality of the second temperature detectors respectively can also detect the temperatures at the medial plantar of foot and the insteps of both feet for the patient, and deliver the alarming signal when the temperature of one of the medial plantar of feet are lower than the temperature of the instep.

In addition, the method for detecting diabetic foot also comprises transmitting an information message, wherein the information message comprises the temperature of the medial plantar of the foot, the temperature of the instep and the alarming signal. Besides, the information message is transmitted via the wireless and saved in a cloud database.

### BRIEF DESCRIPTION OF THE DRAWINGS

Embodiments are illustrated by way of example and not by way of limitation in the figures of the accompanying drawings, in which like references indicate similar elements and in which:
Fig. 1 shows a schematic diagram of one foot;
Fig. 2 shows a stereogram of an early detection electronic device of diabetic foot for lesion in blood circulation according to the first embodiment of the present invention;
Fig. 3 shows a front view of an early detection electronic device of diabetic foot for lesion in blood circulation according to the first embodiment of the present invention;
Fig. 4 shows a circuit block diagram of an early detection electronic device of diabetic foot for lesion in blood circulation according to the first embodiment of the present invention;
Fig. 5 shows a stereogram of an early detection electronic device of diabetic foot for lesion in blood circulation according to the second embodiment of the present invention;
Fig. 6 shows a front view of an early detection electronic device of diabetic foot for lesion in blood circulation according to the second embodiment of the present invention; and

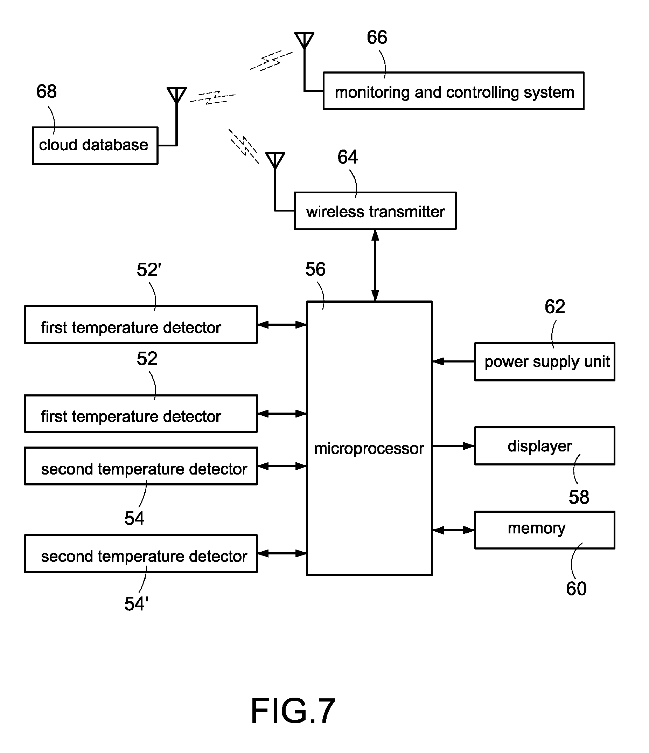

Fig. 7 shows a circuit block diagram of an early detection electronic device of diabetic foot for lesion in blood circulation according to the second embodiment of the present invention.

### DETAILED DESCRIPTION OF PREFERRED EMBODIMENTS

The aforementioned illustrations and following detailed descriptions are exemplary for the purpose of further explaining the scope of the instant disclosure. Other objectives and advantages related to the instant disclosure will be illustrated in the subsequent descriptions and appended drawings.

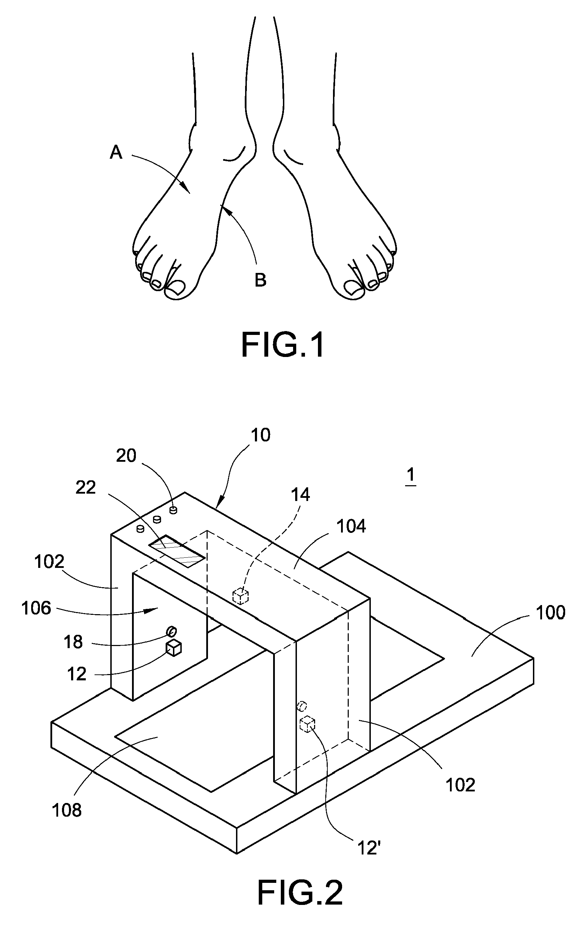

The sign of the diabetic foot is found by the abnormal change of the foot temperature because of a bad blood circulation, in particular, the temperature change of the patient's instep and medial plantar. General speaking, the blood of each tissue in the human body is seriously controlled by the biological needs, and the blood source of the instep is the instep artery. For a normal person, there is less tissue around the instep (as shown in the area A in Fig. 1), so the temperature of instep is relatively low. On the other hand, there is more tissue around the medial plantar (as shown in the area B in Fig. 1), so the temperature of medial plantar is relatively high.

For a diabetic foot patient, the medial plantar artery (at the inner side of the foot bottom) is often to be stuck so that the blood flow would decrease. Thus, the temperature detected at the corresponding surface of the medial plantar artery would be relatively low, which helps to diagnose the diabetic foot and also helps to prevent and suspend the ulcer.

The present invention is to provide an electric device that can detect temperatures at the medial plantar artery (hereafter as medial "plantar") and the instep artery (hereafter as "instep"). Please refer to Fig. 2, Fig. 2 shows a stereogram of an early detection electronic device of diabetic foot for lesion in blood circulation according to the first embodiment of the present invention.

The early detection electronic device 1 of diabetic foot for lesion in blood circulation comprises a base 10, first temperature detectors 12, 12' and a second temperature detector 14. The base 10 comprises a platform part 100, a first arranging part 102 and a second arranging part 104. The platform part 100 is rectangular-plate shaped. The first arranging part 102 is connected to the platform part 100, and the second arranging part 104 is connected to the first arranging part 102.

In this embodiment, the early detection electronic device 1 of diabetic foot for lesion in blood circulation comprises two first arranging parts 102 configured at two opposite sides of the platform part 100 and being perpendicular to the platform part 100. The second arranging part 104 is configured to be parallel with the platform part 100 and be connected to the first arranging parts 102. The platform part 100, the first arranging part 102 and the second arranging part 104 together form a ring-shaped base 10 and a room 106. In practice, the patient's foot is out within the room 106, and the thenar is attached at the foot placing area on the surface of the platform part 100, as shown in Fig. 2.

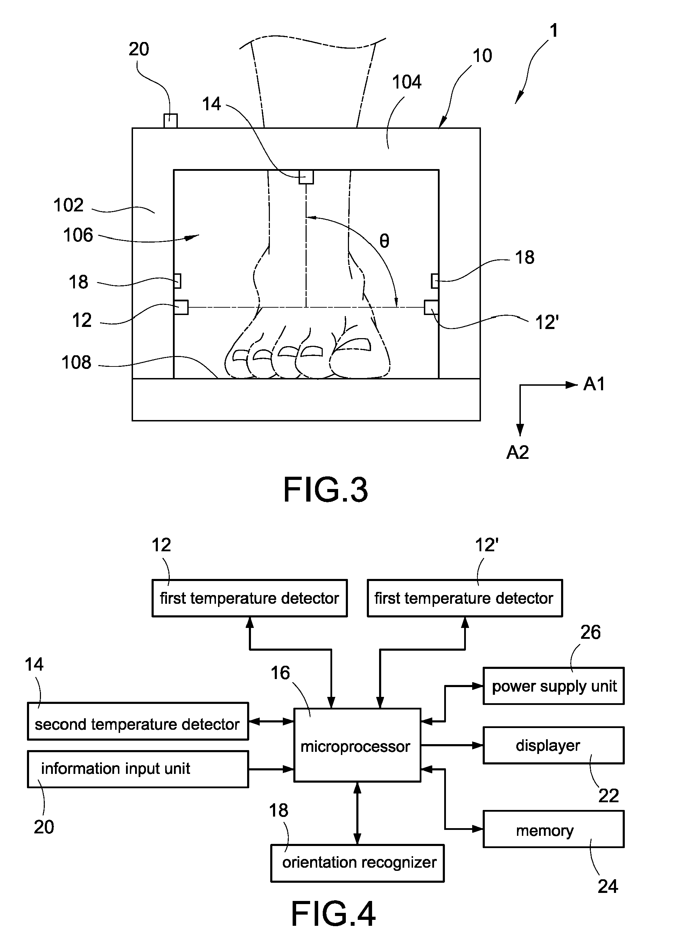

Please refer to Fig. 3, the first temperature detectors 12, 12' are configured at the first arranging part 102. The first temperature detector 12 detects the temperature of the medial plantar of the left foot along the first axis A1, and the first temperature detector 12' detects the temperature of the medial plantar of the right foot along the direction opposite to the first axis A1. The second temperature detector 14 is configured at the second arranging part 104. The second temperature detector 14 detects the temperature of the instep along the second axis A2. There is an included angle θ between the second axis A2 and the first axis A1. In this embodiment, the included angle θ is 90 degrees, but it is not limited thereto.

The early detection electronic device 1 of diabetic foot for lesion in blood circulation further comprises a microprocessor 16 which is electrically connected to the first temperature detectors 12, 12' and a second temperature detector 14, as shown in Fig. 4. The microprocessor 16 controls the first temperature detectors 12, 12' and the second temperature detector 14 to detect the foot temperature for the diabetic patients and to receive the temperature of the medial plantar of the left foot detected by the first temperature detector 12. The first temperature detector 12' detects the temperature of the medial plantar of the right foot, and the second temperature detector 14 detects the temperature of the instep. Particularly, when the temperature of the medial plantar is lower than the temperature of the instep, there is an alarming signal delivered. In other words, the microprocessor 16 compares the temperatures detected by the first temperature detectors 12, 12' and the second temperature detector 14. When the temperatures detected by the first temperature detectors 12, 12' is lower than the temperature detected by the second temperature detector 14, the microprocessor 16 delivers an alarming signal.

Thereby, the user can notice the initial symptoms of the diabetic foot, which helps to prevent and suspend the diabetic foot.

It should be noted that, the early detection electronic device 1 of diabetic foot for lesion in blood circulation can merely comprises a single first temperature detector 12 or 12', so as to detect the temperature of the medial plantar of the left or right foot.

In order to effectively detect the temperature of the medial plantar of the left or right foot, the early detection electronic device 1 of diabetic foot for lesion in blood circulation also comprises an orientation recognizer 18, as shown in Figs. 2-4. The orientation recognizer 18 is electrically connected to the microprocessor 16. The orientation recognizer 18 is configured on the first arranging part 102, as shown in Fig. 3, so as to recognize that the patient's medial plantar is at the left side or the right side of the base 10. The early detection electronic device 1 of diabetic foot for lesion in blood circulation first recognizes that the patient's medial plantar is at the left side or the right side of the base 10 via the orientation recognizer 18, and then transmits the recognizing result to the microprocessor 16. The microprocessor 16 drives the first temperature detectors 12, 12' facing the patient's medial plantar so as to detect the temperature. Thereby, the working efficiency of the early detection electronic device 1 of diabetic foot for lesion in blood circulation can be raised, and further the chances to make misjudgment would decrease. In practice, the orientation recognizer 18 can also be configured on the second arranging part 104.

Moreover, the early detection electronic device 1 of diabetic foot for lesion in blood circulation can also comprise an information input unit 20. As shown in Fig. 4, the information input unit 20 is electrically connected to the microprocessor 16, and the information input unit 20 may be, for example, buttons. The information input unit 20 is not only for the patient to start the early detection electronic device 1 of diabetic foot for lesion in blood circulation, but also for the patient to key in the measurement information, for example, the information about which foot is to be detected, the left one or the right one. As a result, it decreases the chances to make misjudgment for the early detection electronic device 1 of diabetic foot for lesion in blood circulation.

Additionally, the information input unit 20 also provides a function to check the detecting result. For example, the patient's left foot is undertaken a temperature detecting. When the electric device 1 recognizes that the detected foot is the patient's left foot, the patient can input a check message via the information input unit 20. However, when the electric device 1 recognizes that the detected foot is the patient's right foot, the patient can input a correction via the information input unit 20 so as to raise the precision. The information input unit 20 is configured on the platform part 100, the first arranging part 102 or the second arranging part 104. As in Fig. 2, the information input unit 20 is configured on the second arranging part 104 as an example.

The early detection electronic device 1 of diabetic foot for lesion in blood circulation also comprises a displayer 22. As shown in Fig. 2 and Fig. 4, the displayer 22 is configured on the platform part 100 and electrically connected to the microprocessor 16. In this embodiment, the displayer 22 is configured on the second arranging part 104 as an example. The displayer 22 may be, for example, a liquid crystal display, but it is not limited thereto. The displayer 22 is configured to display the alarming sent by the microprocessor 16. Also, the displayer 22 is also configured to show the temperatures detected by the first temperature detectors 12, 12' and the second temperature detector 14. In practice, the information input unit 20 and the displayer 22 can be integrated as a touch screen. The patient can input the measuring information via the touch screen and also can observe the alarming delivered by the microprocessor 16 and the temperatures detected by the first temperature detectors 12, 12' and the second temperature detector 14 via the touch screen.

The early detection electronic device 1 of diabetic foot for lesion in blood circulation further comprises a memory 34 and a power supply unit 26. As shown in Fig. 4, the memory is electrically connected to the microprocessor 16, the first temperature detectors 12, 12' and the second temperature detector 14 so as to save several groups of temperature information detected by the first temperature detectors 12, 12' and the second temperature detector 14. The several groups of temperature information can be shown in tables and diagrams via the displayed 22 or be transmitted to the remote monitoring device via a wireless transmitter. The power supply unit 26 may be, for example, a battery, but it is not limited thereto. The power supply unit 26 is configured to provide power for the operation of the early detection electronic device 1 of diabetic foot for lesion in blood circulation. The memory 24 and the power supply unit 26 may be, for example, configured within the platform part 100.

It should be noted that, the early detection electronic device 1 of diabetic foot for lesion in blood circulation can be combined with other devices which are used to detect human physiology status. For example, the platform part 100 may be a body weight meter, so when the patient is measuring his weight he can have temperature detection at the same time.

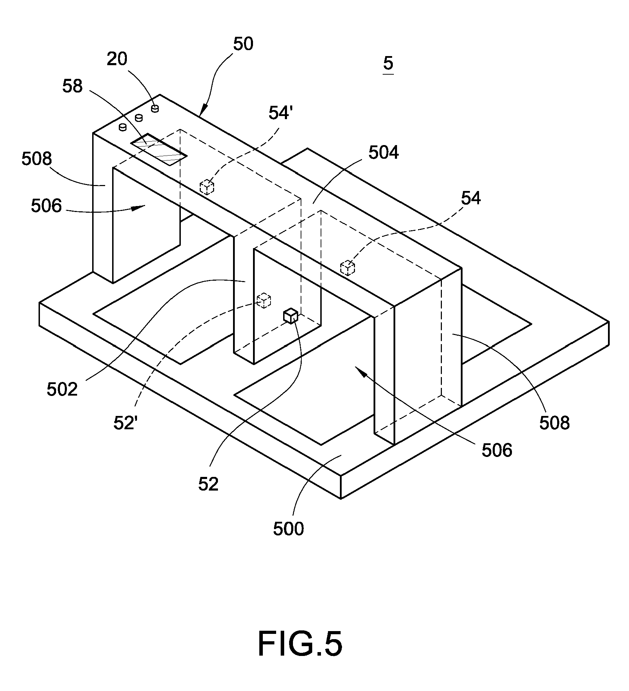

Please refer to Fig. 5, Fig. 5 shows a stereogram of an early detection electronic device of diabetic foot for lesion in blood circulation according to the second embodiment of the present invention. The early detection electronic device 5 of diabetic foot for lesion in blood circulation comprises a base 50, first temperature detectors 52, 52' and second temperature detectors 54, 54'.

The base 50 comprises a platform part 500, a first arranging part 502 and a second arranging part 504. The first arranging part 502 is vertically connected to the central part of the platform part 500. The second arranging part 504 is configured on the platform part 500 in parallel. The first arranging part 502 is connected to the central part of the second arranging part 504 so that the base 50 is I-shaped. The base 50 further comprises sidewall 508 and is connected to the platform 500 and the two opposite sides of the second arranging part 504. Also, the base 50, the platform part 500, the first arranging part 502 and the second arranging part 504 forms a room 506 for the patient to put his feet in.

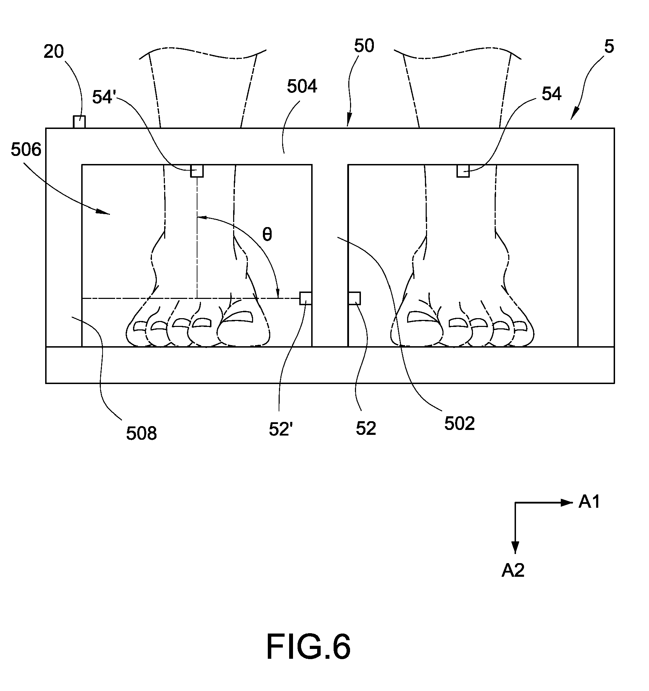

In this embodiment, the early detection electronic device 5 of diabetic foot for lesion in blood circulation comprises two first temperature detectors 52, 52' and two second temperature detectors 54, 54'. The first temperature detectors 52, 52' are configured at the same height on the two opposite surfaces of the first arranging part 502. Also, the first temperature detectors 52, 52' face the sidewall 508 and respectively detect the temperature of the patient's medial plantar along the first axis A1 and the direction opposite the first axis A1, as shown in Fig. 6.

The second temperature detectors 54, 54' are correspondingly configured on the second arranging part 504, so as to detect the temperature of the patient's instep along the second axis A2. There is an included angle θ between the second axis A2 and the first axis A1. In this embodiment, the included angle is 90 degrees, but it is not limited thereto.

In this embodiment, the early detection electronic device 5 of diabetic foot for lesion in blood circulation also comprises a microprocessor 56, a displayer 58, a memory 60 and a power supply unit 62. The first temperature detectors 52, 52', the temperature detectors 54, 54', the displayer 58, the memory 60 and the power supply unit 62 are electrically connected to the microprocessor 56 respectively.

The microprocessor 56 controls the first temperature detectors 52, 52' and the second temperature detectors 54, 54' so as to respectively detect the temperatures of the patient's instep and medial plantar. Also, the microprocessor 56 receives the temperature detected by the first temperature detectors 52, 52' and the second temperature detectors 54, 54'. Moreover, the microprocessor 56 also compares the temperatures of the medial plantar and the instep, and delivers an alarming when the temperature of the medial plantar is lower than the temperature of the instep.

Additionally, the microprocessor 56 also compares the temperatures of the medial plantar and the instep of the patient's two feet, so as to further precisely examine the difference between the temperatures of the medial plantar and the instep of the patient's two feet. Thereby, whether the patient has an initial affection of the diabetic foot can be determined.

The displayer 58 is configured on the platform 500 or the second arranging part 504, and is electrically connected to the microprocessor 56. In this embodiment, the displayer 58 is disposed on the second arranging part 504 as an example. The displayer 58 may be, for example, a liquid crystal display, but it is not limited thereto. The displayer 58 is configured to show the alarming deliver by the microprocessor 56. Also, the displayer 58 can also show the temperature values detected by the first temperature detectors 52, 52' and the second temperature detector 54, 54'. The memory 60 is electrically connected to the microprocessor 56, the first temperature detectors 52, 52' and the second temperature detector 54, 54', so as to save several groups of temperature information detected by the first temperature detectors 52, 52' and the second temperature detector 54, 54'.

The power supply unit 62 may be, for example, a battery, but it is not limited thereto. The power supply unit 62 is configured to provide power for the operation of the early detection electronic device 5 of diabetic foot for lesion in blood circulation. The memory 60 and the power supply unit 62 are, for example, configured within the platform part 500.

The early detection electronic device 5 of diabetic foot for lesion in blood circulation also comprises a wireless transmitter 64 that is electrically connected to the microprocessor 56. The wireless transmitter 64 is configured to wirelessly transmit the alarming signal and the temperatures detected by the first temperature detectors 52, 52' and the second temperature detectors 54, 54' to the remote monitoring and controlling system 66, so as to provide them to the remote department or staff. For example, the alarming signal and the detected temperature are provided to a doctor so as to have an integrated evaluation and an instant monitoring. In addition, the temperature values detected by the first temperature detectors 52, 52' and the second temperature detectors 54, 54' can also be transmitted to and saved in a cloud database 68 via the wireless transmitter 64. Further, the remote department or staff can capture the information from the cloud database 68 so as to have an integrated evaluation. The monitoring and controlling system 66 may be a hand-held device such as a mobile phone or a tablet. The monitoring and controlling system 66 can have a corresponding application installed so as to provide an instant feedback, history information record and other health education information.

The present invention further provide a method for detecting diabetic foot, comprising the following steps: 1) providing a base for putting at least one foot of the patient, at least one first temperature detectors and at least one second temperature detectors are configured on the base, and the first temperature detectors detecting temperature of the medial plantar of foot and the second temperature detectors detecting temperature of the instep; 2) determining whether the temperature of the medial plantar of foot is higher than the temperature of the instep; and 3) delivering an alarming signal when the temperature of the medial plantar of foot is lower than the temperature of the instep.

Moreover, the method for detecting diabetic foot also comprises transmitting an information message, wherein the information message comprises the temperature of the medial plantar of the foot, the temperature of the instep and the alarming signal. Besides, the information message is transmitted via the wireless and saved in a cloud database.

The descriptions illustrated supra set forth simply the preferred embodiments of the instant disclosure; however, the characteristics of the instant disclosure are by no means restricted thereto. All changes, alternations, or modifications conveniently considered by those skilled in the art are deemed to be encompassed within the scope of the instant disclosure delineated by the following claims.

It should be understood that the exemplary embodiments described therein should be considered in a descriptive sense only and not for purposes of limitation. Descriptions of features or aspects within each embodiment should typically be considered as available for other similar features or aspects in other embodiments. While one or more embodiments of the present invention have been described with reference to the figures, it will be understood by those of ordinary skill in the art that various changes in form and details may be made therein without departing from the scope of the present invention as defined by the following claims.

## Claims

1. An early detection electronic device (1, 5) of diabetic foot for lesion in blood circulation, used for detecting temperature of one foot, and the early detection electronic device (1, 5) of diabetic foot for lesion in blood circulation comprising:
a base (10, 50), comprising a platform part (100, 500), at least one first arranging part (102, 502) and one second arranging part (104, 504), the first arranging part (102, 502) connected to the platform part (100, 500), and the second arranging part (104, 504) connected to the first arranging part (102, 502) and parallel with the platform part (100, 500);
at least one first temperature detector (12, 12', 52, 52'), configured horizontally to be at a same height at the first arranging part (102, 502), detecting temperature of the foot along a first axis (A1); and
at least one second temperature detector (14, 54, 54'), disposed on the second arranging part (104, 504), detecting temperature of the foot along a second axis (A2), wherein there is an included angle (θ) between the second axis (A2) and the first axis (A1).

2. The early detection electronic device (1, 5) of diabetic foot for lesion in blood circulation of claim 1, further comprising a microprocessor (16, 56), electrically connected to the first temperature detector (12, 12', 52, 52') and the second temperature detector (14, 54, 54'), the microprocessor (16 ' 56) receiving the temperature detected by the first temperature detector (12, 12', 52, 52') and the second temperature detector (14, 54, 54'), wherein when the temperature of the foot along the first axis is lower than the temperature of the foot along the second axis, the microprocessor (16 ' 56) delivers a warning signal.

3. The early detection electronic device (1, 5) of diabetic foot for lesion in blood circulation of claims 1 or 2, wherein the included angle (θ) is 90 degrees.

4. The early detection electronic device (1, 5) of diabetic foot for lesion in blood circulation of any of the claims 1 to 3, wherein the base comprise two first arranging parts (102, 502) are respectively configured at two opposite sides of the base (10, 50), and the second arranging parts (104, 504) are connected to the first arranging parts (102, 502).

5. The early detection electronic device (1, 5) of diabetic foot for lesion in blood circulation of any of the claims 1 to 4, comprising two first temperature detectors (12, 12', 52, 52') respectively configured on the first arranging part (102, 502), the first temperature detectors (12, 12', 52, 52') detecting the temperature of the foot along the first axis (A1) and the opposite direction of the first axis (A1) respectively.

6. The early detection electronic device (1, 5) of diabetic foot for lesion in blood circulation of any of the claims 1 to 5, further comprising an orientation recognizer (18), configured on the first arranging part (102, 502) or the second arranging part (104, 504), the orientation recognizer (18) electrically connected to the microprocessor (16, 56), and the orientation recognizer (18) used for recognizing whether one medial plantar of the foot is facing the first axis (A1) and driving the first temperature detector (12, 12', 52, 52') facing the first axis (A1) to start temperature detecting.

7. The early detection electronic device (1, 5) of diabetic foot for lesion in blood circulation of any of the claims 1 to 6, wherein the first arranging part (102 ' 502) is connected to a central part of the platform part (100, 500) and the second arranging part (104, 504) so that the base (10, 50) is I-shaped, two first temperature detectors (12, 12', 52, 52') are respectively configured on two opposite surfaces of the first arranging part (102, 502), the first temperature detectors (12, 12', 52, 52') detects the foot temperature along the first axis (A1) and the direction opposite the first axis (A1) respectively, and two second temperature detectors (14, 54, 54') are respectively configured on the second arranging parts (104, 504) on two sides of the first arranging part (102, 502).

8. The early detection electronic device (1, 5) of diabetic foot for lesion in blood circulation of any of the claims 1 to 7, further comprising a displayer (22, 58) and/or a memory (24, 60), electrically connected to the microprocessor (16, 56), the first temperature detector (12, 12', 52, 52') and the second temperature detector (14, 54, 54').

9. The early detection electronic device (1, 5) of diabetic foot for lesion in blood circulation of any of the claims 1 to 8, further comprising a power supply unit (26, 62), electrically connected to the microprocessor (16, 56), the power supply unit (26, 62) used for provide power for operation of the early detection electronic device (1, 5) of diabetic foot for lesion in blood circulation.

10. The early detection electronic device (1, 5) of diabetic foot for lesion in blood circulation of any of the claims 1 to 9, further comprising a wireless transmitter (64), electrically connected to the microprocessor (16, 56), the wireless transmitter (64) configured to wirelessly transmit the temperatures detected by the first temperature detector (12, 12', 52, 52') and the second temperature detector (14, 54, 54') to a monitoring and controlling system (66).

11. The early detection electronic device (1, 5) of diabetic foot for lesion in blood circulation of any of the claims 1 to 10, wherein the monitoring and controlling system (66) is a hand-held device, and an application is installed in the monitoring and controlling system so as to provide instant feedback, history information record and health education.

12. The early detection electronic device (1, 5) of diabetic foot for lesion in blood circulation of any of the claims 1 to 11, further comprising an information input unit (20), electrically connected to the microprocessor (16, 56).

13. A method for detecting diabetic foot, comprising:
providing a base (10, 50) for putting at least one foot of the patient, at least one first temperature detector (12, 12', 52, 52') and at least one second temperature detector (14, 54, 54') are configured on the base (10, 50), and the first temperature detector (12, 12', 52, 52') detecting temperature of the medial plantar of foot and the second temperature detector (14, 54, 54') detecting temperature of the instep;
determining whether the temperature of the medial plantar of foot is higher than the temperature of the instep; and
delivering an alarming signal when the temperature of the medial plantar of foot is lower than the temperature of the instep.

14. The method of claim 13, wherein the plurality of the first temperature detectors (12, 12', 52, 52') and the plurality of the second temperature detectors (14, 54, 54') respectively detects the temperatures at the medial plantar of foot and the insteps of both feet for the patient, and delivers the alarming signal when the temperature of one of the medial plantar of feet are lower than the temperature of the instep.

15. The method of claim 13 or 14 , further comprising transmitting an information message, wherein the information message comprises the temperature of the medial plantar of the foot, the temperature of the instep and the alarming signal,
wherein the information message is preferably transmitted via the wireless and saved in a cloud database (68).
